# EUROPEAN PATENT APPLICATION

(11) **EP 1 335 012 A2**
(43) Date of publication of application: **13.08.2003**
(21) Application number: 03000388.3
(22) Date of filing: 10.01.2003
(51) Int. Cl.: C09K 5/18, F25D 5/02, A61F 7/10

(54) **Formulation of a substantially granular product for obtaining instantaneous ice bags**

(30) Priority: 08.02.2002 IT VR20020011
(71) Applicant: Phyto Performance Italia S.r.l., 35030 Veggiano Padova (IT)
(72) Inventor: De Pieri, Sara, 35030 Rubano (PD) (IT)
(74) Representative: Savi, Alberto

(57) **Abstract**

The new proposed formulation solves the problems due to the combustibility of ammonium nitrate. In practice, ammonium nitrate is replaced with another material which has never been used in these applications so far. This material offers the same results as ammonium nitrate but it is not inflammable, nor is it explosive.

In general, this new formulation of a granular product for the obtention of instantaneous ice bags is based on the use of granular urea, which is the base material of which this product is made.

## Description

The present invention proposes a new formulation of a substanzially granular product for the obtention of instantaneous ice bags.

This product is very useful in the curative first aid therapy to cure bruises, strains, hematomas, muscular sprains, headache, toothache, body overheating, traumas and blows in general.

This product may be used in the sports and medical field. In any case, this product may be used whenever an immediate cold application is necessary (cryotherapy).

In general, this new formulation is used to obtain a sealed bag. Once the contents of the bag are activated, the bag becomes cold immediately and remains cold for many minutes.

As is known, in the sports and medical field as well as in the field of free time activities there are provided first aid applications to remove or at least to relieve the first painful symptons of a trauma such as a bruise, a strain, a hematoma, a muscular sprain, a headache, a toothache, a body overheating or a blow.

In general, the said known first aid applications or products are made of ice. As is well-known, ice is able to relieve traumas and pains of the human body. Ice is available in different forms such as ice spray bottle, ice bag to be kept in the freezer and sealed bag containing instantaneous ice.

The sealed bag containing instantaneous ice consists of a bag in which there are two components to be activated. The activation is provoked by beating the sealed bag with the hand (a firm stroke) so that an inner bubble containing liquid is broken and the bag become very cold immediately.

In general, the components used in the said instantaneous ice bags are ammonium nitrate and water. Once these components have combined, they produce cold for about 30-40 minutes.

However, there is the following drawback in the utilization of such components: ammonium nitrate is an explosive material. As a matter of fact, ammonium nitrate is utilized in the preparation of explosives and therefore, this material is very dangerous, especially in case ammonium nitrate is stored in great quantities.

The aim of the present invention is to conceive and carry out a new formulation to solve the problems due to the utilization of ammonium nitrate and therefore, ammonium nitrate is replaced with another material which has never been used in the said applications so far and offers the same results as ammonium nitrate without being inflammable. An immediate advantage of the proposed solution is obvious: a complete elimination of danger, which makes any safety measures unnecessary. On the contrary, safety measures were necessary to store the known comburent for the obtention of instantaneous ice.

The aforesaid aim and advantages are reached according to the present invention through a new formulation of a substantially granular product for the obtention of instantaneous ice bags, characterized in that the base material of this product is urea which is dissolved in water and generates ice cold and that the urea contents are dosed in variable percentages depending on the size of the bag.

The bag according to the present invention contains two components as follows:
1. A granular product made of urea;
2. Water or other liquid. This part may be contained in a further bag which is put in the first bag.

After breaking the inner bag containing water, water mixes with urea and a reaction takes place and produces ice cold.

For instance, the contents of an instantaneous ice bag may be as follows:
- g 100-250 urea
- small nylon bag containing g 140 water.

After breaking the small nylon bag with a stroke, urea dissolves in water and generates cold.

Concerning the possible formulations, the most representative formulations are the two following ones:
Big bag (cm 24 x 14.5): g 0.200 granular urea;
   g 0.140 water in small nylon bag.
Small bag (cm 18 x 15): g 0.175 granular urea;
   g 0.140 water in small nylon bag.

In addition, a Table is supplied below as an example. This Table reports a possible configuration of the specifics as well as chemical and physical properties of the granular urea:

| Product: Granular Urea | | | |
|---|---|---|---|
| Paramaters | Unit of Measure | Values | Analytical Method |
| Formaldehyde | % p.p. | 0.2-0.35 | M.I. F004 |
| Humidity | " | 0.3 max | M.I. F002 |
| Total Nitrogen | " | 46 min | S.G.U. 180 5/8/86 |
| Bi-ureic acid salt | " | 1.2 max | S.G.U. 180 5/8/86 |
| Sulphates | p.p.m. | 10 max | M.I. F019 |
| Borden Test | - | 6.6 min | M.I. F017 |
| Coore | APHA | < 20 | M.I. F005 |
| Bulk Density | Kg/m³ | 750-820 | EN 1236 |
| Granular Measure | | | M.I. F006 |
| > 5 mm | % p.p. | 5 max | |
| < 2 mm | " | 2 max | |

As it can be seen, the so-described formulation removes the problem due to the high inflammability of ammonium nitrate because ammonium nitrate is replaced with urea which is an inert material as regards combustion.

## Claims

1. Formulation of a substantially granular product for the obtention of an instantaneous ice bag, **characterized in that** the base material of this product is urea which is dissolved in water and produces ice cold and that urea contents are dosed in various percentages depending on the size of the bag.

2. Formulation for the obtention of an instantaneous ice bag, **characterized in that** the bag contains two parts, namely, a granular product made of urea, and water or other liquid which may be contained in a further bag to be put in the first bag and that after breaking the inner bag containing water, water mixes with urea and a reaction takes place and generates ice cold.

3. Formulation for the obtention of an instantaneous ice bag, **characterized in that** the contents of an instantaneous bag may be 100-250 g urea and a small nylon bag containing 140 g water, and after breaking the small nylon bag with a stroke, urea dissolves in water and generates cold.
